# DEMANDE DE BREVET EUROPEEN

(11) **EP 3 263 092 A1**
(43) Date de publication de la demande: **03.01.2018**
(21) Numéro de dépôt: 17179430.8
(22) Date de dépôt: 03.07.2017
(51) Int. Cl.: A61K 8/25, A61K 8/67, A61Q 19/08, A61K 8/92

(54) **FORMULATION COSMETIQUE COMPRENANT UN PRECURSEUR LIPOSOLUBLE DE SILANOL ET UN UN ESTER D'ACIDE GRAS DE VITAMINE C**

(30) Priorité: 02.07.2016 FR 1601045
(71) Demandeur: Piron, Estelle, 73490 La Ravoire (FR)
(72) Inventeur: Piron, Estelle, 73490 La Ravoire (FR)
(74) Mandataire: Tripoz, Inès

(57) **Abrégé**

L'invention concerne une formulation cosmétique comprenant une phase lipophile, caractérisée en ce que ladite phase lipophile comprend :
- au moins une huile ;
- au moins un ester d'acide gras de vitamine C ou une vitamine C liposoluble ; et
- au moins un précurseur liposoluble de silanol ou un silicium liposoluble ;
le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile étant supérieur ou égal à 1,5.

## Description

L'invention concerne le domaine technique des soins cosmétiques de la peau et des phanères.

L'invention est relative à une formulation cosmétique comprenant une phase lipophile, caractérisée en ce que ladite phase lipophile comprend au moins une huile et au moins deux actifs cosmétiques liposolubles : au moins une vitamine C liposoluble et au moins un silicium liposoluble, le rapport molaire entre ladite au moins une vitamine C liposoluble et ledit au moins un silicium liposoluble dans ladite phase lipophile étant supérieur ou égal à 1,5.

La formulation cosmétique selon l'invention est par exemple destinée à traiter la peau et les phanères.

L'invention est également relative à un procédé de préparation d'une formulation cosmétique selon l'invention, ainsi qu'à certaines utilisations de la formulation cosmétique selon l'invention.

L'invention est enfin relative à un kit comprenant une formulation cosmétique selon l'invention, et un dispositif d'application de type roller.

La vitamine C est une vitamine hydrophile jouant un rôle important dans le métabolisme de l'être humain et de nombreux autres mammifères.

La vitamine C est notamment connue pour ses très bonnes propriétés antioxydantes (protection vis-à-vis des UVA, UVB, vieillissement cutané photo-induit). La vitamine C intervient également en synergie avec la vitamine E, protégeant les membranes lipidiques cellulaires de la peroxydation. La vitamine C est également connue comme agent stimulant la croissance des fibroblastes, générant ainsi une production accrue de collagène et d'élastine. En outre, elle favorise la production du collagène de par son rôle d'incorporation de la proline au sein du collagène en cours de formation.

Il est connu que la vitamine C est relativement peu stable en milieu aqueux, de plus elle est rapidement dégradée à l'air libre et/ou à la lumière, ce qui constitue une grande difficulté dans le cadre de son incorporation en des quantités significatives, dans les formulations cosmétiques.

Un certain nombre de dérivés de la vitamine C existent, incluant des dérivés hydrophiles (dont par exemple l'ascorbyle phosphate de magnesium, l'ascorbyle phosphate de sodium, etc.) et des dérivés lipophiles (dont par exemple le tétraisopalmitate d'ascorbyle, le palmitate d'ascorbyle, le dipalmitate d'ascorbyle, etc.). Dans le cadre de la présente invention, les dérivés liposolubles de la vitamine C sont appelés « vitamines C liposolubles ».

Le silicium est l'élément chimique de numéro atomique 14, et de symbole Si. Le silicium est notamment connu pour son effet inhibiteur ou activateur de nombreux mécanismes biochimiques, de structuration/protection de nombreuses macromolécules de soutien (élastine, collagène, protéoglycane, etc.), comme potentialisateur d'agents thérapeutiques, comme détoxifiant, comme agent favorisant le processus de minéralisation dans l'ossification, ou encore comme composant structurel et défenseur de différents tissus conjonctifs tels que les os, le cartilage, le derme et l'aorte. En résumé, le silicium a un rôle dans la fermeté et l'élasticité des tissus conjonctifs, et notamment du derme. Cependant, avec l'âge, sa concentration dans le derme et autres tissus conjonctifs diminue, participant, entre autres, à l'affaiblissement de la peau et à l'apparition des rides.

Les atomes de silicium peuvent notamment être apportés par des molécules organiques, parfois appelées « siliciums organiques », ces molécules libèrent le silicium, par exemple au contact de la peau.

Les atomes de silicium peuvent entrer dans la formule chimique de composés hydrophiles (comme par exemple le mannuronate de methylsilanol, le silanol, le silanetriol ou le siloxanetriol) ou lipophiles (comme par exemple, le dioleyl tocopheryl methylsilanol CAS 130986-04-8, le dimethyl oxobenzo dioxasilane CAS 17902-57-7),

Dans le cadre de la présente invention, un certain nombre de définitions sont données ci-après.

Dans le cadre de la présente invention, les composés liposolubles comprenant du silicium sont appelés « siliciums liposolubles ».

On appelle « silicium liposoluble », un silicium liposoluble étant soluble à l'échelle moléculaire dans un milieu huileux, formant ainsi une solution huileuse.

Ces « siliciums liposolubles » sont des « précurseurs liposolubles de silanol », c'est-à-dire qu'ils sont susceptibles par hydrolyse de libérer des silanols hydrosolubles de formule SiH_{4-*n*}(OH)*ₙ* (*n* = 1, 2, 3, 4), composés très instables qui vont être métabolisés dans les couches hydrophiles du derme.

On appelle « huile » un ingrédient cosmétique lipophile pouvant être, à température ambiante (25°C), liquide (huile liquide) ou solide (huile concrète). Une huile liquide commence à s'écouler sous son propre poids en moins d'une minute à température ambiante (25 °C), à l'inverse d'une huile concrète.

On appelle « vitamine C liposoluble », une vitamine C étant soluble à l'échelle moléculaire dans un milieu huileux, formant ainsi une solution huileuse. Il peut notamment s'agir du tetraisopalmitate d'ascorbyle (CAS 183476-82-6), du dipalmitate d'ascorbyle ou encore du palmitate d'ascorbyle (CAS 137-66-6).

Dans la présente invention, il s'agit d'esters (mono ou polyesters d'acides gras de vitamine C).

On appelle « huile naturelle », une huile issue directement d'une source naturelle, elle peut notamment être animale ou végétale, et peut notamment être obtenue par pression à froid d'un végétal. De préférence, l'huile est issue d'une source végétale. L'huile naturelle est différente de l'« huile estérifiée », cette dernière ayant subi une estérification.

On appelle « rapport molaire » le rapport entre le nombre de moles d'une première entité chimique et le nombre de moles d'une seconde entité chimique.

On appelle « concentration molaire » le nombre de moles d'une entité chimique dans une quantité déterminée de formulation. La concentration molaire est exprimée en « moles/l ».

On appelle « ingrédient cosmétique liposoluble additionnel » un ingrédient cosmétique n'étant ni une vitamine C liposoluble, ni un silicium liposoluble, ni l'au moins une huile.

On appelle « actif cosmétique liposoluble additionnel » un ingrédient cosmétique liposoluble additionnel utilisé en tant qu'ingrédient actif.

On appelle « entité chimique » une molécule (comme par exemple la vitamine C ou encore les vitamines C liposolubles) ou un atome (comme par exemple l'atome de Si). Une entité chimique est soit un atome unique, soit plusieurs atomes liés par des liaisons covalentes.

On appelle « entités chimiques différentes » des entités chimiques non liées par des liaisons covalentes.

On appelle « formulation exempte d'eau », une formulation comprenant par exemple moins de 0,5 % d'eau par rapport à la masse totale de ladite formulation (c'est-à-dire moins de 0,5 g d'eau pour 100 g de formulation).

On appelle « dispositif d'application de type roller » un dispositif comprenant un cylindre comportant une pluralité de micro-aiguilles. Le cylindre est destiné à être appliqué sur la peau, les micro-aiguilles créant ainsi des micro-perforations de la peau qui favorisent la pénétration des actifs.

Dans le cadre de la présente demande, on appelle composé « liposoluble » un composé qualifié selon la Pharmacopée Européenne de « soluble », « facilement soluble » à « très soluble » ; c'est-à-dire nécessitant moins de 30 ml d'huile pour dissoudre 1 g de substance.

Ces composés sont insolubles dans l'eau. On appelle dans le cadre de la présente demande composé « insoluble dans l'eau » un composé qualifié selon la Pharmacopée Européenne de « très peu soluble » ou « pratiquement insoluble » dans l'eau ; c'est-à-dire nécessitant plus de 1000 ml d'eau pour dissoudre un gramme de produit.

En résumé, s'agissant des deux entités chimiques que sont la vitamine C et le silicium, la vitamine C est fortement antioxydante et favorise la synthèse de collagène et d'élastine, et le silicium intervient dans le pontage de ces macromolécules, afin de créer un tissu ferme, élastique et résistant.

L'association de ces deux entités chimiques dans des formulations destinées à être appliquées sur la peau, et sur les phanères, comprenant une phase lipophile présente donc un véritable intérêt et la demanderesse a mis en évidence de façon surprenante que les effets étaient optimaux lorsque le rapport molaire entre la vitamine C liposoluble et le silicium liposoluble dans ladite phase lipophile était supérieur ou égal à 1,5.

Dans la demande WO 2010/146142 au nom de HDS LIMITED, il est question d'une méthode et d'une formulation ayant pour but de réduire ou d'inhiber l'action délétère des pigments pour cheveux sur la peau. Le tetraisopalmitate d'ascorbyle (vitamine C liposoluble), ainsi que le dioleyl tocopheryl methylsilanol (silicium liposoluble) sont cités dans une longue liste de composés antioxydants. La demande ne comprend aucun exemple, ni de mode de réalisation associant ces deux composés.

Dans la demande WO 98/44904 au nom de THE BOOTS COMPANY PLC, un certain nombre de molécules comprenant des atomes de silicium sont citées, en particulier le dimethyl oxobenzo dioxasilane et le dioleyl tocopheryl methylsilanol (page 7), en outre le palmitate d'ascorbyle est cité page 11. Les exemples de cette demande portent uniquement sur des émulsions, et à la fois le silicium et la vitamine C sont contenus dans la phase aqueuse. Les composés liposolubles simplement cités ne sont pas inclus dans les formulations des exemples, qui sont des émulsions.

Par ailleurs, la formulation commerciale Ascorbosilane C^{®} a été développée par la société EXSYMOL, il s'agit d'un dérivé conjugué de vitamine C et de silicium (vitamine C et silicium liés de manière covalente). Dans le produit Ascorbosilane C^{®}, la vitamine C et le silicium sont isomolaires. De plus, cette préparation est hydrophile, de sorte que lorsque la vitamine C est libérée, cette dernière est quasi-immédiatement dégradée.

La société EXSYMOL a également décrit un certain nombre de composés, les demandes WO 96/10574 et WO 96/10575 y sont relatives. Par exemple, un des composés (lipophile) cité et exemplifié est le diméthylsilyl-2,3,5,6-ascorbate. Il est déductible de la formule chimique présente en page 9 de la demande WO 96/10575 qu'une mole d'acide ascorbique est libérée lorsque deux moles de silicium sont libérées.

Le but des inventions décrites dans ces demandes est de fournir des composés à base de silicium possédant des liaisons biologiquement hydrolysables au contact de la peau. Dans ces demandes sont illustrées les difficultés que rencontre l'homme de l'art lorsqu'il doit obtenir des formulations stables et efficaces comprenant à la fois de la vitamine C et du silicium (nécessité d'apporter des précurseurs, etc.).

De manière surprenante, il a été mis en évidence par la demanderesse que l'association dans une phase lipophile d'au moins une huile et d'au moins deux actifs : au moins une vitamine C liposoluble et au moins un silicium liposoluble (ou au moins un ester d'acide gras de vitamine C et au moins un précurseur liposoluble de silanol), le rapport molaire entre ladite au moins une vitamine C liposoluble et ledit au moins un silicium liposoluble (ou entre ledit au moins un ester d'acide gras de vitamine C et ledit au moins un précurseur liposoluble de silanol) dans ladite phase lipophile étant supérieur ou égal à 1,5, permettait d'obtenir des formulations cosmétiques :
- ayant des propriétés dermatologiques améliorées ;
- très stables ;
- faciles à formuler ;
- ayant une cinétique/une séquence d'effets optimale.

En particulier, ces propriétés sont améliorées lorsque le rapport molaire entre ladite au moins une vitamine C liposoluble et ledit au moins un silicium liposoluble (ou entre ledit au moins un ester d'acide gras de vitamine C et ledit au moins un précurseur liposoluble de silanol) est de préférence supérieur à 1,5, de préférence supérieur à 2, de préférence supérieur à 3, ou compris entre 1,5 et 40, de préférence entre 1,5 et 35, de préférence entre 1,5 et 20.

De plus, la formulation ne contient que très peu d'allergènes (sauf ceux éventuellement présents dans les huiles essentielles).
En particulier, la composition qualitative et quantitative des formulations ne change pas, même après stockage pendant 5 mois à température ambiante.

En particulier, il n'y a pas de nécessité d'inclure de tensioactif, la formulation est huileuse, et peut comprendre un nombre limité de constituants (par exemple seulement trois constituants : l'huile et les deux actifs liposolubles). En outre, la préparation peut notamment consister en un simple mélange, à température ambiante, préservant ainsi la vitamine C, particulièrement sensible à la chaleur. Par ailleurs, il n'y a pas de nécessité d'inclure d'agent conservateur.

C'est de manière particulièrement surprenante que la demanderesse a observé des effets synergiques entre le précurseur liposoluble de silanol ou le silicium liposoluble et l'ester d'acide gras de vitamine C ou la vitamine C liposoluble, notamment lorsque la vitamine C liposoluble est en quantité relative (par rapport au silicium liposoluble) relativement importante, c'est-à-dire lorsque le rapport molaire entre ladite au moins une vitamine C liposoluble et ledit au moins un silicium liposoluble est de préférence supérieur à 1,5, de préférence supérieur à 2, de préférence supérieur à 3, ou compris entre 1,5 et 40, de préférence entre 1,5 et 35, de préférence entre 1,5 et 20.

Sans vouloir être lié par une quelconque explication, il est supposé que la vitamine C est impliquée dans plus d'actions au niveau de la peau (antioxydant, régénération de la vitamine E, action sur les fibroblastes pour booster la synthèse du collagène et de l'élastine, incorporation de la proline dans le collagène, contrôle/régulation de la mélanine, etc.) que le silicium. Il est également supposé que la vitamine C est consommée en tant qu'antioxydant (UVA, UVB, fumée, stress, etc.), et que le rôle antioxydant de la vitamine C est très important et nécessite donc une concentration initiale plus importante par rapport à celle du silicium. Il est observé ensuite l'action complémentaire de la vitamine C et du silicium sur les cellules de la peau, la peau est durablement raffermie.

Le terme « micro-needling » désigne l'ensemble des techniques utilisant des micro-aiguilles qui micro-perforent la peau. Les micro perforations réalisées vont permettre une pénétration accrue des principes actifs appliqués sur la peau (facteur 100 à 1000 selon les auteurs dans la littérature) et vont, selon leur longueur et leur profondeur de pénétration, générer une induction/stimulation mécanique sur les cellules de la peau (fibroblastes) provoquant une régénération des fibres de collagène et d'élastine du derme (principe de la CIT : Collagen Induction Therapy), ainsi qu'une activation des systèmes capillaire et sanguin, avec néovascularisation. Des longueurs d'aiguilles de 0,2 mm resteront dans l'épiderme et en surface du derme superficiel, avec une activation faible voire inexistante des fibroblastes (présents dans le derme), alors que des longueurs d'aiguilles de 0,3 à 0,5 mm atteindront le derme superficiel et engendreront une CIT au sein de ce dernier. Avec des longueurs d'aiguilles supérieures à 0,5mm (0,75 ou 1mm), le derme profond sera atteint et on aura alors une CIT sur toute l'épaisseur de la peau, à savoir sur les 2 parties du derme.

Associer cette technique de micro-needling aux formulations cosmétiques selon l'invention permet d'agir sur les fibroblastes en 3 étapes / à 3 niveaux l'induction mécanique favorisant leur activité de synthèse de collagène et d'élastine, activité qui sera boostée par la présence de vitamine C et de silicium, ce dernier servira en outre à garantir une bonne structure de l'ensemble de néo-fibres ainsi formées.

Dans le domaine du micro-needling, on trouve notamment les « dispositifs d'application de type roller », qui sont des dispositifs comprenant un cylindre comportant une pluralité de micro-aiguilles. Le cylindre est destiné à être appliqué sur la peau, les micro-aiguilles créant ainsi des micro-perforations de la peau qui favorisent la pénétration des actifs.

Associer cette technique de micro-needling au moyen d'un dispositif d'application de type roller aux formulations cosmétiques selon l'invention permet, outre les avantages liés au micro-needling, de générer une desquamation accrue de la peau, qui pourrait s'apparenter à une desquamation superficielle, l'association de ce traitement mécanique avec les formulations cosmétiques selon l'invention va permettre de régénérer de façon efficace et rapide la couche lipidique du stratum corneum.

L'invention concerne une formulation cosmétique comprenant une phase lipophile, caractérisée en ce que ladite phase lipophile comprend :
- au moins une huile ;
- au moins un ester d'acide gras de vitamine C ; et
- au moins un précurseur liposoluble de silanol ;
le rapport molaire entre ledit au moins un ester d'acide gras de vitamine C et ledit au moins un précurseur liposoluble de silanol dans ladite phase lipophile étant supérieur ou égal à 1,5.

Dans un mode de réalisation la formulation cosmétique selon l'invention est caractérisée en ce que le au moins un ester d'acide gras de vitamine C est une vitamine C liposoluble ; et le au moins un précurseur liposoluble de silanol et un silicium liposoluble.

L'invention concerne une formulation cosmétique comprenant une phase lipophile, caractérisée en ce que ladite phase lipophile comprend :
- au moins une huile ;
- au moins une vitamine C liposoluble ; et
- au moins un silicium liposoluble ;
le rapport molaire entre ladite au moins une vitamine C liposoluble et ledit au moins un silicium liposoluble dans ladite phase lipophile étant supérieur ou égal à 1,5.

Dans un mode de réalisation, la formulation cosmétique selon l'invention est caractérisée en ce que le rapport molaire entre ledit au moins un ester d'acide gras de vitamine C et ledit au moins un précurseur liposoluble de silanol dans ladite phase lipophile est supérieur ou égal à 2.

Dans un mode de réalisation, la formulation cosmétique selon l'invention est caractérisée en ce que le rapport molaire entre ledit au moins un ester d'acide gras de vitamine C et ledit au moins un précurseur liposoluble de silanol dans ladite phase lipophile est supérieur ou égal à 3.

Dans un mode de réalisation, la formulation cosmétique selon l'invention est caractérisée en ce que le rapport molaire entre ledit au moins un ester d'acide gras de vitamine C et ledit au moins un précurseur liposoluble de silanol dans ladite phase lipophile est compris entre 1,5 et 40.

Dans un mode de réalisation, la formulation cosmétique selon l'invention est caractérisée en ce que le rapport molaire entre ledit au moins un ester d'acide gras de vitamine C et ledit au moins un précurseur liposoluble de silanol dans ladite phase lipophile est compris entre 1,5 et 35.

Dans un mode de réalisation, la formulation cosmétique selon l'invention est caractérisée en ce que le rapport molaire entre ladite au moins une vitamine C liposoluble et ledit au moins un silicium liposoluble dans ladite phase lipophile est supérieur ou égal à 2.

Dans un mode de réalisation, la formulation cosmétique selon l'invention est caractérisée en ce que le rapport molaire entre ladite au moins une vitamine C liposoluble et ledit au moins un silicium liposoluble dans ladite phase lipophile est supérieur ou égal à 3.

Dans un mode de réalisation, la formulation cosmétique selon l'invention est caractérisée en ce que le rapport molaire entre ladite au moins une vitamine C liposoluble et ledit au moins un silicium liposoluble dans ladite phase lipophile est compris entre 1,5 et 40.

Dans un mode de réalisation, la formulation cosmétique selon l'invention est caractérisée en ce que le rapport molaire entre ladite au moins une vitamine C liposoluble et ledit au moins un silicium liposoluble dans ladite phase lipophile est compris entre 1,5 et 35.

Dans un mode de réalisation, la formulation cosmétique selon l'invention est caractérisée en ce que ladite au moins une vitamine C liposoluble ou le au moins un ester d'acide gras de vitamine C est un ester d'acide gras choisi dans le groupe constitué par les acides constitués d'une chaîne alkyle insaturée ou saturée, ramifiée ou non ramifiée, comprenant de 6 à 50 carbones, 6 à 20 atomes de carbones, de préférence 6 à 10 atomes de carbones.

Dans un mode de réalisation, les acides gras sont choisis dans le groupe constitué par les acides gras linéaires.

Dans un mode de réalisation, les acides gras linéaires sont choisis dans le groupe constitué par l'acide caproïque, l'acide oenanthique, l'acide caprylique, l'acide caprique, l'acide nonanoïque, l'acide décanoïque, l'acide undécanoïque, l'acide dodécanoïque, l'acide palmitique, l'acide stéarique, l'acide arachidique, l'acide béhénique, l'acide tricosanoïque, l'acide lignocérique, l'acide heptacosanoïque, l'acide octacosanoïque et l'acide mélissique.

Dans un mode de réalisation, les acides gras sont choisis dans le groupe constitué par les acides gras insaturés.

Dans un mode de réalisation, les acides gras insaturés sont choisis dans le groupe constitué par l'acide myristoléique, l'acide palmitoléique, l'acide oléique, l'acide élaidique, l'acide linoléique, l'acide alpha-linoléique, l'acide arachidonique, l'acide eicosapentaenoïque, l'acide érucique et l'acide docosahexaénoïque.

Dans un mode de réalisation, les acides gras sont choisis dans le groupe constitué par les acides de la bile et leurs dérivés.

Dans un mode de réalisation, les acides de la bile et leurs dérivés sont choisis dans le groupe constitué par l'acide cholique, l'acide déhydrocholique, l'acide désoxycholique et l'acide chénodésoxycholique.

Dans un mode de réalisation, la formulation cosmétique selon l'invention est caractérisée en ce que ledit au moins un ester d'acide gras de vitamine C ou ladite au moins une vitamine C liposoluble est un ester d'acide ascorbique et d'acide palmitique, telle que le palmitate d'ascorbyle, le dipalmitate d'ascorbyle ou encore le tétraisopalmitate d'ascorbyle.

Dans un mode de réalisation, la formulation cosmétique selon l'invention est caractérisée en ce que le rapport molaire entre ledit au moins un ester d'acide gras de vitamine C et ledit au moins un précurseur liposoluble de silanol ou ladite au moins une vitamine C liposoluble et ledit au moins un silicium liposoluble dans ladite phase lipophile est compris entre 1,5 et 20.

Dans un mode de réalisation, la formulation cosmétique selon l'invention est caractérisée en ce que la concentration molaire en l'au moins un ester d'acide gras de vitamine C ou l'au moins une vitamine C liposoluble est supérieure à 0,001 mol/l de ladite phase lipophile.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile.

Dans un mode de réalisation, la formulation cosmétique selon l'invention est caractérisée en ce que la concentration molaire en l'au moins un ester d'acide gras de vitamine C ou l'au moins une vitamine C liposoluble est d'environ 0,01 mol/l de ladite phase lipophile.

Dans un mode de réalisation, la formulation cosmétique selon l'invention est caractérisée en ce que la concentration molaire en l'au moins un précurseur liposoluble de silanol ou l'au moins un silicium liposoluble est supérieure à 0,0005 mol/l de ladite phase lipophile.

Dans un mode de réalisation, la formulation cosmétique selon l'invention est caractérisée en ce que la concentration molaire en l'au moins un précurseur liposoluble de silanol ou l'au moins un silicium liposoluble est comprise entre 0,0005 et 0,01 mol/l de ladite phase lipophile.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C et ledit au moins un précurseur liposoluble de silanol ou entre ladite au moins une vitamine C liposoluble et ledit au moins un silicium liposoluble dans ladite phase lipophile est supérieur ou égal à 1,5.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C et ledit au moins un précurseur liposoluble de silanol ou entre ladite au moins une vitamine C liposoluble et ledit au moins un silicium liposoluble dans ladite phase lipophile est supérieur ou égal à 2.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre Iledit au moins un ester d'acide gras de vitamine C ou ladite au moins une vitamine C liposoluble et ledit au moins un précurseur liposoluble de silanol ou ledit au moins un silicium liposoluble dans ladite phase lipophile est supérieur ou égal à 3.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur ou ledit au moins un silicium liposoluble dans ladite phase lipophile est compris entre 1,5 et 40.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 35.

Dans un mode de réalisation, la formulation cosmétique selon l'invention est caractérisée en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 20.

Dans un mode de réalisation, la formulation cosmétique selon l'invention est caractérisée en ce que ledit au moins un ester d'acide gras de vitamine C ou ladite au moins une vitamine C liposoluble est choisi dans le groupe constitué du palmitate d'ascorbyle, du dipalmitate d'ascorbyle, du tétraisopalmitate d'ascorbyle, et de leurs mélanges.

L'hydratation est très irrégulière selon les couches constituant la peau. Le derme contient environ 80% d'eau et en remontant vers l'épiderme la teneur en eau diminue. Elle est voisine de 60% au niveau de la couche basale pour n'être plus que de 10% à 20% environ en surface, au niveau de la couche cornée. La surface de la peau est donc très majoritairement lipophile, et des actifs de nature hydrophile nécessiteront l'usage de tensioactifs pour permettre leur pénétration jusqu'à l'épiderme sous-jacent. Des composés lipophiles n'auront pas cette difficulté, en revanche il leur sera plus difficile de pénétrer au sein de l'épiderme puis du derme, de plus en plus hydrophiles.

La nature lipophile de la phase comprenant ledit au moins un ester d'acide gras de vitamine C ou ladite vitamine C liposoluble permet une meilleure pénétration de ladite vitamine C liposoluble dans la peau, à travers la couche cornée lipophile. Par ailleurs, une fois que ledit au moins un ester d'acide gras de vitamine C ou ladite vitamine C a pénétré dans la peau, la biodisponibilité de la vitamine C est idéale, car elle est convertie en vitamine C, hydrosoluble, sous l'action d'estérases et d'autres enzymes de la peau.

Dans un mode de réalisation, la formulation cosmétique selon l'invention est caractérisée en ce que ledit au moins un ester d'acide gras de vitamine C ou ladite au moins une vitamine C liposoluble est le palmitate d'ascorbyle.

Dans un mode de réalisation, la formulation cosmétique selon l'invention est caractérisée en ce que ledit au moins un ester d'acide gras de vitamine C ou ladite au moins une vitamine C liposoluble est le dipalmitate d'ascorbyle.

Dans un mode de réalisation, la formulation cosmétique selon l'invention est caractérisée en ce que ledit au moins un ester d'acide gras de vitamine C ou ladite au moins une vitamine C liposoluble est le tétraisopalmitate d'ascorbyle.

Dans un mode de réalisation, la formulation cosmétique selon l'invention est caractérisée en ce que l'au moins un précurseur liposoluble de silanol ou ledit au moins un silicium liposoluble est choisi dans le groupe constitué des précurseurs liposolubles de silanols.

Dans un mode de réalisation, la formulation cosmétique selon l'invention est caractérisée en ce que l'au moins un précurseur liposoluble de silanol ou ledit au moins un silicium liposoluble est choisi dans le groupe constitué du dioleyl tocopheryl methylsilanol, du dimethyl oxobenzo dioxasilane, et de leurs mélanges.

Le dimethyl oxobenzo dioxasilane est parfois associé à l'ethylhexyl palmitate, par exemple dans la composition commerciale Pro-DSB^{®}.

Dans un mode de réalisation, la formulation cosmétique selon l'invention est caractérisée en ce que l'au moins un précurseur liposoluble de silanol ou ledit au moins silicium liposoluble est le dioleyl tocopheryl methylsilanol.

Dans un mode de réalisation, la formulation cosmétique selon l'invention est caractérisée en ce que l'au moins un précurseur liposoluble de silanol ou ledit au moins silicium liposoluble est le dimethyl oxobenzo dioxasilane.

Ces précurseurs lipophiles de silanol, en raison à la fois de leur caractère lipophile et de l'hydrolyse qu'ils subissent en présence d'eau, vont pénétrer facilement dans la peau (bonne pénétration de la couche cornée majoritairement lipophile), et libérer facilement du silicium une fois dans la peau (hydrolyse permettant le détachement d'atomes de silicium et leur mise à disposition immédiate).

Dans un mode de réalisation, la formulation cosmétique selon l'invention est caractérisée en ce que ledit au moins un ester d'acide gras de vitamine C ou ladite au moins une vitamine C liposoluble est le tétraisopalmitate d'ascorbyle et ledit au moins un silicium liposoluble est le dimethyl oxobenzo dioxasilane.

Dans un mode de réalisation, la formulation cosmétique selon l'invention est caractérisée en ce que ledit au moins un ester d'acide gras de vitamine C ou ladite au moins une vitamine C liposoluble est le tétraisopalmitate d'ascorbyle et ledit au moins un silicium liposoluble est le dioleyl tocopheryl methylsilanol.

Dans un mode de réalisation, la formulation cosmétique selon l'invention est caractérisée en ce que ledit au moins un ester d'acide gras de vitamine C ou ladite au moins une vitamine C liposoluble est le palmitate d'ascorbyle et ledit au moins un silicium liposoluble est le dimethyl oxobenzo dioxasilane.

Dans un mode de réalisation, la formulation cosmétique selon l'invention est caractérisée en ce que ledit au moins un ester d'acide gras de vitamine C ou ladite au moins une vitamine C liposoluble est le palmitate d'ascorbyle et ledit au moins un silicium liposoluble est le dioleyl tocopheryl methylsilanol.

Dans un mode de réalisation, la formulation cosmétique selon l'invention est caractérisée en ce que ledit au moins un ester d'acide gras de vitamine C ou ladite au moins une vitamine C liposoluble est le dipalmitate d'ascorbyle et ledit au moins un silicium liposoluble est le dimethyl oxobenzo dioxasilane.

Dans un mode de réalisation, la formulation cosmétique selon l'invention est caractérisée en ce que ledit au moins un ester d'acide gras de vitamine C ou ladite au moins une vitamine C liposoluble est le dipalmitate d'ascorbyle et ledit au moins un silicium liposoluble est le dioleyl tocopheryl methylsilanol.

Dans un mode de réalisation, la formulation cosmétique selon l'invention est caractérisée en ce que l'au moins une huile est choisie dans le groupe constitué des huiles liquides, des huiles concrètes, et de leurs mélanges.

Dans un mode de réalisation, la formulation cosmétique selon l'invention est caractérisée en ce que l'au moins une huile est une huile liquide.

Dans un mode de réalisation, la formulation cosmétique selon l'invention est caractérisée en ce que l'au moins une huile est une huile concrète.

Dans un mode de réalisation, la formulation cosmétique selon l'invention est caractérisée en ce que l'au moins une huile est une huile choisie dans le groupe constitué des huiles naturelles, des huiles estérifiées, et de leurs mélanges.

Dans un mode de réalisation, la formulation cosmétique selon l'invention est caractérisée en ce que l'au moins une huile est une huile naturelle.

Dans un mode de réalisation, la formulation cosmétique selon l'invention est caractérisée en ce que l'au moins une huile est une huile naturelle et est choisie dans le groupe constitué de l'huile de tournesol, de l'huile de macadamia, de l'huile de noyaux d'abricot, de l'oléine de karité, de l'huile de sésame, de l'huile de germe de blé, de l'huile de colza, de l'huile d'olive, de l'huile de jojoba, de l'huile de nigelle, de l'huile de rose musquée, de l'huile de grains de café vert et de leurs mélanges.

Dans un mode de réalisation, la formulation cosmétique selon l'invention est caractérisée en ce que l'au moins une huile est une huile estérifiée.

Dans un mode de réalisation, la formulation cosmétique selon l'invention est caractérisée en ce que l'au moins une huile est une huile estérifiée et est choisie dans le groupe constitué des esters d'acides gras.

Dans un mode de réalisation, la formulation cosmétique selon l'invention est caractérisée en ce que l'au moins une huile est une huile estérifiée et est choisie dans le groupe constitué des esters de l'acide caprylique.

Dans un mode de réalisation, la formulation cosmétique selon l'invention est caractérisée en ce que l'au moins une huile est une huile estérifiée et est choisie dans le groupe constitué des caprylic/capric triglycerides, du dicaprylyl carbonate, des coco-caprylate caprates, et de leurs mélanges.

Dans un mode de réalisation, la formulation cosmétique selon l'invention est caractérisée en ce que ladite phase lipophile comprend en outre au moins un ou plusieurs ingrédient(s) cosmétique(s) liposoluble(s) additionnel(s).

Dans un mode de réalisation, la formulation cosmétique selon l'invention est caractérisée en ce que ladite phase lipophile comprend en outre au moins un ou plusieurs ingrédient(s) cosmétique(s) liposoluble(s) additionnel(s) choisi(s) dans le groupe constitué par les adoucissants, les antioxydants, les opacifiants, les stabilisants, les émollients, les silicones, les charges, les polymères, les colorants, ou tout autre ingrédient habituellement utilisé en cosmétique, en particulier pour la fabrication de formulations cosmétiques huileuses, et leurs mélanges.

Dans un mode de réalisation, la formulation cosmétique selon l'invention est caractérisée en ce que ladite phase lipophile comprend en outre au moins un ou plusieurs ingrédient(s) cosmétique(s) liposoluble(s) additionnel(s) choisi(s) dans le groupe des adoucissants.

Dans un mode de réalisation, la formulation cosmétique selon l'invention est caractérisée en ce que ladite phase lipophile comprend en outre au moins un ou plusieurs ingrédient(s) cosmétique(s) liposoluble(s) additionnel(s) choisi(s) dans le groupe des antioxydants.

Dans un mode de réalisation, la formulation cosmétique selon l'invention est caractérisée en ce que ladite phase lipophile comprend en outre au moins un ou plusieurs ingrédient(s) cosmétique(s) liposoluble(s) additionnel(s) choisi(s) dans le groupe des antioxydants qui est le tocophérol.

Dans un mode de réalisation, la formulation cosmétique selon l'invention est caractérisée en ce que ladite phase lipophile comprend en outre au moins un ou plusieurs ingrédient(s) cosmétique(s) liposoluble(s) additionnel(s) choisi(s) dans le groupe des opacifiants.

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s).

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s) choisi(s) dans le groupe constitué des vitamines A et E.

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s) choisi(s) dans le groupe constitué des filtres solaires liposolubles.

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s) choisi(s) dans le groupe constitué des agents de bronzage et/ou de brunissage artificiel de la peau (agents autobronzants).

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s) choisi(s) dans le groupe constitué du coenzyme Q10, du rétinyl palmitate, du tocophéryl acétate, de l'extrait de criste marine, de l'extrait de la fleur de spilanthes acmella africaine, et de leurs mélanges.

Dans un mode de réalisation, la formulation cosmétique selon l'invention est caractérisée en ce que ladite phase lipophile comprend en outre au moins du coenzyme Q10.

Dans un mode de réalisation, la formulation cosmétique selon l'invention est caractérisée en ce que ladite phase lipophile comprend en outre au moins du rétinyl palmitate.

Dans un mode de réalisation, la formulation cosmétique selon l'invention est caractérisée en ce que ladite phase lipophile comprend en outre au moins du tocophéryl acétate.

Dans un mode de réalisation, la formulation cosmétique selon l'invention est caractérisée en ce que ladite phase lipophile comprend en outre au moins de l'extrait de Criste marine.

Dans un mode de réalisation, la formulation cosmétique selon l'invention est caractérisée en ce que ladite phase lipophile comprend en outre au moins de l'extrait de la fleur de *spilanthes acmella* africaine.

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s) choisi(s) dans le groupe constitué des pigments ou des nanopigments.

Dans un mode de réalisation, la formulation cosmétique selon l'invention est caractérisée en ce que ladite phase lipophile comprend en outre au moins un ou plusieurs actif(s) cosmétique(s) liposoluble(s) additionnel(s) choisi(s) dans le groupe constitué des pigments ou des nanopigments d'oxydes métalliques enrobés ou non comme par exemple des nanopigments d'oxyde de titane de fer, de zinc, de zirconium ou de cérium qui sont tous des agents photoprotecteurs connus agissant par blocage physique du rayonnement UV, et de leurs mélanges.

Dans un mode de réalisation, la formulation cosmétique selon l'invention est caractérisée en ce que ladite au moins une vitamine C liposoluble et ledit au moins un silicium liposoluble appartiennent à des entités chimiques différentes.

Dans un mode de réalisation, la formulation cosmétique selon l'invention est caractérisée en ce que ladite formulation est constituée par ladite phase lipophile.

Dans un mode de réalisation, la formulation cosmétique selon l'invention est caractérisée en ce que ladite formulation est constituée par ladite phase lipophile et ladite phase lipophile est exempte d'eau.

L'invention concerne un procédé de préparation d'une formulation cosmétique selon l'invention comprenant au moins les étapes suivantes de formation d'une phase lipophile :
1) on dispose d'au moins une huile, d'au moins un ester d'acide gras de vitamine C ou d'au moins une vitamine C liposoluble et d'au moins un précurseur liposoluble de silanol ou d'au moins un silicium liposoluble ;
2) l'au moins une huile, ledit au moins un ester d'acide gras de vitamine C ou l'au moins une vitamine C liposoluble et ledit au moins un précurseur liposoluble de silanol ou l'au moins un silicium liposoluble sont mélangés ;
le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile étant supérieur ou égal à 1,5.

Dans un mode de réalisation, le procédé de préparation d'une formulation cosmétique selon l'invention est caractérisé en ce que l'étape 2 est réalisée à température ambiante.

Dans un mode de réalisation, lors de l'étape 2, l'au moins un ester d'acide gras de vitamine C ou l'au moins une vitamine C et/ou l'au moins un précurseur liposoluble de silanol ou l'au moins un silicium liposoluble est(sont) ajouté(s) dans l'au moins une huile.

Dans un mode de réalisation, le procédé de préparation d'une formulation cosmétique selon l'invention est caractérisé en ce que le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est supérieur ou égal à 2.

Dans un mode de réalisation, le procédé de préparation d'une formulation cosmétique selon l'invention est caractérisé en ce que le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est supérieur ou égal à 3.

Dans un mode de réalisation, le procédé de préparation d'une formulation cosmétique selon l'invention est caractérisé en ce que le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 40.

Dans un mode de réalisation, le procédé de préparation d'une formulation cosmétique selon l'invention est caractérisé en ce que le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 35.

Dans un mode de réalisation, le procédé de préparation d'une formulation cosmétique selon l'invention est caractérisé en ce que le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 20.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est supérieure à 0,001 mol/l de ladite phase lipophile.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est d'environ 0,01 mol/l de ladite phase lipophile.

Dans un mode de réalisation, le procédé de préparation d'une formulation cosmétique selon l'invention est caractérisé en ce que la concentration molaire en l'au moins un précurseur liposoluble de silanol ou l'au moins un silicium liposoluble est supérieure à 0,0005 mol/l de ladite phase lipophile.

Dans un mode de réalisation, le procédé de préparation d'une formulation cosmétique selon l'invention est caractérisé en ce que la concentration molaire en l'au moins un précurseur liposoluble de silanol ou l'au moins un silicium liposoluble est comprise entre 0,0005 et 0,01 mol/l de ladite phase lipophile.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est supérieur ou égal à 1,5.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est supérieur ou égal à 2.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est supérieur ou égal à 3.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 40.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 35.

Dans un mode de réalisation, le procédé de préparation d'une formulation cosmétique selon l'invention est caractérisé en ce que la concentration molaire en l'au moins un ester d'acide gras de vitamine C ou l'au moins une vitamine C liposoluble est comprise entre 0,001 et 0,2 mol/l de ladite phase lipophile, la concentration molaire en l'au moins un précurseur liposoluble de silanol ou l'au moins un silicium liposoluble est comprise entre 0,0005 et 0,01 mol/l de ladite phase lipophile, et le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile est compris entre 1,5 et 20.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est choisie dans le groupe constitué du palmitate d'ascorbyle, du dipalmitate d'ascorbyle, du tétraisopalmitate d'ascorbyle, et de leurs mélanges.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le palmitate d'ascorbyle.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le dipalmitate d'ascorbyle.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le tétraisopalmitate d'ascorbyle.

Dans un mode de réalisation, le procédé de préparation d'une formulation cosmétique selon l'invention est caractérisé en ce que ledit au moins un précurseur liposoluble de silanol ou ledit au moins un silicium liposoluble est choisi dans le groupe constitué du dioleyl tocopheryl methylsilanol, du dimethyl oxobenzo dioxasilane, et de leurs mélanges.

Dans un mode de réalisation, le procédé de préparation d'une formulation cosmétique selon l'invention est caractérisé en ce que ledit au moins un précurseur liposoluble de silanol ou ledit au moins un silicium liposoluble est le dioleyl tocopheryl methylsilanol.

Dans un mode de réalisation, le procédé de préparation d'une formulation cosmétique selon l'invention est caractérisé en ce que ledit au moins un précurseur liposoluble de silanol ou ledit au moins un silicium liposoluble est le dimethyl oxobenzo dioxasilane.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le tétraisopalmitate d'ascorbyle et ledit au moins un silicium liposoluble est le dimethyl oxobenzo dioxasilane.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le tétraisopalmitate d'ascorbyle et ledit au moins un silicium liposoluble est le dioleyl tocopheryl methylsilanol.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le palmitate d'ascorbyle et ledit au moins un silicium liposoluble est le dimethyl oxobenzo dioxasilane.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le palmitate d'ascorbyle et ledit au moins un silicium liposoluble est le dioleyl tocopheryl methylsilanol.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le dipalmitate d'ascorbyle et ledit au moins un silicium liposoluble est le dimethyl oxobenzo dioxasilane.

Dans un mode de réalisation, le procédé de préparation d'une formulation cosmétique selon l'invention est caractérisé en ce que ledit au moins un ester d'acide gras de vitamine C ou ladite au moins une vitamine C liposoluble est le dipalmitate d'ascorbyle et ledit au moins un silicium liposoluble est le dioleyl tocopheryl methylsilanol.

Dans un mode de réalisation, le procédé de préparation d'une formulation cosmétique selon l'invention est caractérisé en ce que l'au moins une huile est choisie dans le groupe constitué des huiles liquides, des huiles concrètes, et de leurs mélanges.

Dans un mode de réalisation, le procédé de préparation d'une formulation cosmétique selon l'invention est caractérisé en ce que l'au moins une huile est une huile liquide.

Dans un mode de réalisation, le procédé de préparation d'une formulation cosmétique selon l'invention est caractérisé en ce que l'au moins une huile est une huile concrète.

Dans un mode de réalisation, le procédé de préparation d'une formulation cosmétique selon l'invention est caractérisé en ce que l'au moins une huile est une huile choisie dans le groupe constitué des huiles naturelles, des huiles estérifiées, et de leurs mélanges.

Dans un mode de réalisation, le procédé de préparation d'une formulation cosmétique selon l'invention est caractérisé en ce que l'au moins une huile est une huile naturelle.

Dans un mode de réalisation, le procédé de préparation d'une formulation cosmétique selon l'invention est caractérisé en ce que l'au moins une huile est une huile naturelle végétale.

Dans un mode de réalisation, le procédé de préparation d'une formulation cosmétique selon l'invention est caractérisé en ce que l'au moins une huile est une huile naturelle végétale et est choisie dans le groupe constitué de l'huile de tournesol, de l'huile de macadamia, de l'huile de noyaux d'abricot, de l'oléine de karité, de l'huile de sésame, de l'huile de germe de blé, de l'huile de colza, de l'huile d'olive, de l'huile de jojoba, de l'huile de nigelle, de l'huile de rose musquée, de l'huile de grains de café vert et de leurs mélanges.

Dans un mode de réalisation, le procédé de préparation d'une formulation cosmétique selon l'invention est caractérisé en ce que l'au moins une huile est une huile estérifiée.

Dans un mode de réalisation, le procédé de préparation d'une formulation cosmétique selon l'invention est caractérisé en ce que l'au moins une huile est une huile estérifiée et est choisie dans le groupe constitué des esters d'acides gras.

Dans un mode de réalisation, le procédé de préparation d'une formulation cosmétique selon l'invention est caractérisé en ce que l'au moins une huile est une huile estérifiée et est choisie dans le groupe constitué des esters de l'acide caprylique.

Dans un mode de réalisation, le procédé de préparation d'une formulation cosmétique selon l'invention est caractérisé en ce que l'au moins une huile est une huile estérifiée et est choisie dans le groupe constitué des caprylic/capric triglycerides, du dicaprylyl carbonate, des coco-caprylate caprates, et de leurs mélanges.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs ingrédient(s) cosmétique(s) liposoluble(s) additionnel(s).

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs ingrédient(s) cosmétique(s) liposoluble(s) additionnel(s) choisi(s) dans le groupe constitué par les adoucissants, les antioxydants, les opacifiants, les stabilisants, les émollients, les silicones, les charges, les polymères, les colorants, ou tout autre ingrédient habituellement utilisé en cosmétique, en particulier pour la fabrication de formulations cosmétiques huileuses, et leurs mélanges.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs ingrédient(s) cosmétique(s) liposoluble(s) additionnel(s) choisi(s) dans le groupe des adoucissants.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs ingrédient(s) cosmétique(s) liposoluble(s) additionnel(s) choisi(s) dans le groupe des antioxydants.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs ingrédient(s) cosmétique(s) liposoluble(s) additionnel(s) choisi(s) dans le groupe des antioxydants qui est le tocophérol.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs ingrédient(s) cosmétique(s) liposoluble(s) additionnel(s) choisi(s) dans le groupe des opacifiants.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s).

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s) choisi(s) dans le groupe constitué des vitamines A et E.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s) choisi(s) dans le groupe constitué des filtres solaires liposolubles.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s) choisi(s) dans le groupe constitué des agents de bronzage et/ou de brunissage artificiel de la peau (agents autobronzants).

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s) choisi(s) dans le groupe constitué du coenzyme Q10, du rétinyl palmitate, du tocophéryl acétate, de l'extrait de criste marine, de l'extrait de la fleur de *spilanthes acmella* africaine, et de leurs mélanges.

Dans un mode de réalisation, le procédé de préparation d'une formulation cosmétique selon l'invention est caractérisé en ce qu'il comprend en outre au moins une étape d'ajout dans ladite phase lipophile d'au moins du coenzyme Q10.

Dans un mode de réalisation, le procédé de préparation d'une formulation cosmétique selon l'invention est caractérisé en ce qu'il comprend en outre au moins une étape d'ajout dans ladite phase lipophile d'au moins du rétinyl palmitate.

Dans un mode de réalisation, le procédé de préparation d'une formulation cosmétique selon l'invention est caractérisé en ce qu'il comprend en outre au moins une étape d'ajout dans ladite phase lipophile d'au moins du tocophéryl acétate.

Dans un mode de réalisation, le procédé de préparation d'une formulation cosmétique selon l'invention est caractérisé en ce qu'il comprend en outre au moins une étape d'ajout dans ladite phase lipophile d'au moins de l'extrait de criste marine.

Dans un mode de réalisation, le procédé de préparation d'une formulation cosmétique selon l'invention est caractérisé en ce qu'il comprend en outre au moins une étape d'ajout dans ladite phase lipophile d'au moins de l'extrait de la fleur de spilanthes acmella africaine.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s) choisi(s) dans le groupe constitué des pigments ou des nanopigments.

Dans un mode de réalisation, le procédé de préparation d'une formulation cosmétique selon l'invention est caractérisé en ce qu'il comprend en outre au moins une étape d'ajout dans ladite phase lipophile d'au moins un ou plusieurs actif(s) cosmétique(s) liposoluble(s) additionnel(s) choisi(s) dans le groupe constitué des pigments ou des nanopigments d'oxydes métalliques enrobés ou non comme par exemple des nanopigments d'oxyde de titane de fer, de zinc, de zirconium ou de cérium qui sont tous des agents photoprotecteurs connus agissant par blocage physique du rayonnement UV, et de leurs mélanges.

Dans un mode de réalisation, le procédé de préparation d'une formulation cosmétique selon l'invention est caractérisé en ce que ladite au moins une vitamine C liposoluble et ledit au moins un silicium liposoluble appartiennent à des entités chimiques différentes.

Dans un mode de réalisation, le procédé de préparation d'une formulation cosmétique selon l'invention est caractérisé en ce que ladite formulation est constituée par ladite phase lipophile.

Dans un mode de réalisation, le procédé de préparation d'une formulation cosmétique selon l'invention est caractérisé en ce que ladite formulation est constituée par ladite phase lipophile et ladite phase lipophile est exempte d'eau.

L'invention concerne également les utilisations d'une formulation cosmétique selon l'invention.

L'invention concerne l'utilisation d'une formulation cosmétique selon l'invention pour stimuler les fibroblastes.

L'invention concerne également une formulation cosmétique, destinée à être utilisée pour stimuler les fibroblastes.

L'invention concerne l'utilisation d'une formulation cosmétique selon l'invention pour protéger l'épiderme humain.

L'invention concerne également une formulation cosmétique, destinée à être utilisée pour protéger l'épiderme humain.

L'invention concerne l'utilisation d'une formulation cosmétique selon l'invention pour protéger l'épiderme humain.

L'invention concerne également une formulation cosmétique, destinée à être utilisée pour protéger l'épiderme humain.

L'invention concerne l'utilisation d'une formulation cosmétique selon l'invention pour protéger les cheveux.

L'invention concerne également une formulation cosmétique, destinée à être utilisée pour protéger les cheveux.

L'invention concerne l'utilisation d'une formulation cosmétique selon l'invention en tant que formulation anti-âge.

L'invention concerne également une formulation cosmétique, destinée à être utilisée en tant que formulation anti-âge.

L'invention concerne l'utilisation d'une formulation cosmétique selon l'invention en tant que formulation d'amélioration de la qualité de la peau.

L'invention concerne également une formulation cosmétique, destinée à être utilisée en tant que formulation d'amélioration de la qualité de la peau.

L'invention concerne l'utilisation d'une formulation cosmétique selon l'invention en tant que formulation d'amélioration de la qualité des cheveux.

L'invention concerne également une formulation cosmétique, destinée à être utilisée en tant que formulation d'amélioration de la qualité des cheveux.

L'invention concerne également une formulation cosmétique, destinée à être appliquée au moyen d'une technique de micro-needling.

L'invention concerne également une formulation cosmétique, destinée à être utilisée dans une technique de micro-needling.

Dans un mode de réalisation, la longueur des micro-aiguilles est comprise entre 0,2 et 0,5 mm.

Dans un mode de réalisation, la longueur des micro-aiguilles est comprise entre 0,3 et 0,75 mm.

Dans un mode de réalisation, la longueur des micro-aiguilles est comprise entre 0,75 et 1 mm.

L'invention concerne un kit cosmétique comprenant au moins :
- une formulation cosmétique selon l'invention ;
- un dispositif d'application de type roller.

### EXEMPLES

Dans les exemples suivants, certains actifs sont incorporés au moyen de compositions commerciales les comprenant mais n'étant pas constituées uniquement de ces actifs. Les explications ci-après permettent de calculer les teneurs en les différents actifs comme l'au moins une vitamine C liposoluble (Cosphaderm AP^{®}, VC-IP^{®}) ou l'au moins un silicium liposoluble (Liposiliol C^{®}, Pro-DSB^{®}).

La composition commerciale VC-IP^{®} est composée d'environ 100% (m/m) de tétraisopalmitate d'ascorbyle.

Le tétraisopalmitate d'ascorbyle ayant une masse molaire de 1129,7 g/mol, les correspondances entre les concentrations massiques et les concentrations molaires sont (par exemple) les suivantes :
- 0,1% (m/m) de VC-IP^{®} équivaut à 0,00088 mol/l de ladite phase lipophile ;
- 0,5% (m/m) de VC-IP^{®} équivaut à 0,0044 mol/l de ladite phase lipophile ;
- 5% (m/m) de VC-IP^{®} équivaut à 0,044 mol/l de ladite phase lipophile ;
- 10% (m/m) VC-IP^{®} équivaut à 0,088 mol/l de ladite phase lipophile.

La composition commerciale Cosphaderm AP^{®} est composée d'environ 100% (m/m) de palmitate d'ascorbyle.

Le palmitate d'ascorbyle ayant une masse molaire de 414,5 g/mol, les correpondances entre les concentrations massiques et les concentrations molaires sont (par exemple) les suivantes :
- 0,5% (m/m) de Cosphaderm AP^{®} équivaut à 0,012 mol/l de ladite phase lipophile ;
- 5% (m/m) de Cosphaderm AP^{®} équivaut à 0,12 mol/l de ladite phase lipophile ;
- 10% (m/m) de Cosphaderm AP^{®} équivaut à 0,24 mol/l de ladite phase lipophile.

La composition commerciale Pro-DSB^{®} est composée d'environ 1,45% (m/m) d'atomes de silicium.

Le silicium ayant masse molaire de 28 g/mol, les correspondances entre les concentrations massiques et les concentrations molaires sont (par exemple) les suivantes :
- 0,1% (m/m) de Pro-DSB^{®} équivaut à 0,000518 mol/l de silicium dans ladite phase lipophile ;
- 1% (m/m) de Pro-DSB^{®} équivaut à 0,00518 mol/l de silicium dans ladite phase lipophile ;
- 2% (m/m) de Pro-DSB^{®} équivaut à 0,01036 mol/l de silicium dans ladite phase lipophile.

La composition commerciale Liposiliol C^{®} est composée d'environ 0,15% (m/m) d'atomes de silicium.

Le silicium ayant masse molaire de 28 g/mol, les correspondances entre les concentrations massiques et les concentrations molaires sont (par exemple) les suivantes :
- 1% (m/m) de Liposiliol C^{®} équivaut à 0,00053 mol/l de silicium dans ladite phase lipophile ;
- 6% (m/m) de Liposiliol C^{®} équivaut à 0,00318 mol/l de silicium dans ladite phase lipophile ;
- 10% (m/m) de Liposiliol C^{®} équivaut à 0,0053 mol/l de silicium dans ladite phase lipophile.

### Exemple 1 : Formulations cosmétiques selon l'invention.

### Préparation des formulations cosmétiques selon l'invention F1 à F3 :

L'ensemble des ingrédients cosmétiques choisis dans le groupe constitué des huiles et huiles estérifiées sont pesés ensemble et homogénéisés à température ambiante pendant 10 minutes.

Le tétraisopalmitate d'ascorbyle (nom commercial VC-IP^{®}), le dioleyl tocopheryl methylsilanol (nom commercial Liposiliol C^{®}) et le tocophérol sont ensuite ajoutés, toujours à température ambiante, et homogénéisés avec l'ensemble pendant 10 minutes.

Les formulations cosmétiques F1 à F3 selon l'invention sont détaillées dans le tableau 1 ci-dessous :

**Tableau 1 : formulations cosmétiques F1 à F3 selon l'invention**

| | **Compositions quantitatives % m/m** | | |
|---|---|---|---|
| Constituants | F1 | F2 | F3 |

| **Vitamine C et silicium liposolubles** | | | |
|---|---|---|---|
| VC-IP^{®} (tétraisopalmitate d'ascorbyle) | 3 | 2 | 10 |
| Liposiliol C^{®} (dioleyl tocopheryl methylsilanol) | 3 | 5 | 6 |
| **Rapport molaire vitamine C/silicium** | **16,6** | **6,6** | **27,5** |

| **Huiles** | | | |
|---|---|---|---|
| Huile Macadamia | 20 | 32,85 | / |
| Huile Noyaux d'abricot | 10 | 20 | / |
| Huile Tournesol | 20 | 40 | / |
| Oleine de karite | 10 | / | / |

| **Huiles estérifiées** | | | |
|---|---|---|---|
| Caprylic capric triglycerides | 20 | / | 48,9 |
| Coco-caprylate caprate | / | / | 20 |
| Dicaprylyl carbonate | 13,8 | / | 15 |

| **Ingrédient cosmétique additionnel** | | | |
|---|---|---|---|
| Tocophérol | 0,2 | 0,15 | 0,1 |

### Préparation des formulations cosmétiques F4 à F7 selon l'invention :

L'ensemble des ingrédients cosmétiques choisis dans le groupe constitué des huiles et huiles estérifiées sont pesés ensemble et homogénéisés à température ambiante pendant 10 minutes.

Le tétraisopalmitate d'ascorbyle (nom commercial VC-IP^{®}), le dimethyl oxobenzo dioxasilane (nom commercial Pro-DSB^{®}) et le tocophérol sont ensuite ajoutés, toujours à température ambiante, et homogénéisés avec l'ensemble pendant 10 minutes.

Les formulations réalisées F4 à F7 sont détaillées dans le tableau 2 ci-dessous :

| | **Compositions quantitatives % m/m** | | | |
|---|---|---|---|---|
| Constituants | F4 | F5 | F6 | F7 |

| **Vitamine C et silicium liposolubles** | | | | |
|---|---|---|---|---|
| VC-IP^{®} (tétraisopalmitate d'ascorbyle) | 1 | 8 | 1,5 | 0,6 |
| Pro-DSB^{®} (dimethyl oxobenzo dioxasilane) | 0,2 | 1 | 0,5 | 0,5 |
| **Rapport molaire vitamine C / silicium** | **8,5** | **13,5** | **5,1** | **2,04** |

| **Huiles** | | | | |
|---|---|---|---|---|
| Huile germe de blé | 5 | / | / | 2 |
| Huile Macadamia | 10 | 45 | / | 15 |
| Huile Noyaux d'abricot | / | / | / | 6,7 |
| Huile de ricin | / | / | / | 8 |
| Huile Sésame | 5 | 15,85 | / | 8 |
| Huile Tournesol | 15 | 30 | 40 | 15 |
| Oléine de karité | 8,6 | / | / | / |

| **Huiles estérifiées** | | | | |
|---|---|---|---|---|
| Caprylic capric triglycerides | 25 | / | 32,85 | 20 |
| Coco-caprylate caprate | 15 | / | / | 10 |
| Dicaprylyl carbonate | 15 | / | 25 | 14 |

| **Ingrédient cosmétique additionnel** | | | | |
|---|---|---|---|---|
| Tocophérol | 0,2 | 0,15 | 0,15 | 0,2 |

### Tableau 2 : formulations cosmétiques F4 à F7 selon l'invention

### Exemple 2 : Stabilité des formulations cosmétiques selon l'invention.

Les formulations cosmétiques F1, F3, F5 et F7 selon l'invention ont été conservées à température ambiante (22°C +/- 3°C), en flacons plastique (PP) transparents fermés, pendant 5 mois.

A la fin de chaque mois pendant les 5 mois, des observations visuelles ont été menées, portant sur les aspects suivants :
- transparence ;
- persistence de la couleur jaune clair ;
- absence de dépôt, particule ou tout autre changement d'aspect ;
- absence de changement d'odeur.

Aucun changement n'a été observé pendant ces 5 mois, prouvant la stabilité des formulations cosmétiques F1, F3, F5 et F7 selon l'invention dans ces conditions de conservation.

### Exemple 3 : Formulations cosmétiques selon l'invention incluant un actif cosmétique liposoluble additionnel.

### Préparation des formulations cosmétiques F8 à F13 selon l'invention :

L'ensemble des ingrédients cosmétiques choisis dans le groupe constitué des huiles naturelles ou estérifiées sont pesés ensemble et homogénéisés à température ambiante pendant 10 minutes.

Le tétraisopalmitate d'ascorbyle (nom commercial VC-IP^{®}) ou le palmitate d'ascorbyle (nom commercial Cosphaderm AP®), le dimethyl oxobenzo dioxasilane (nom commercial Pro-DSB^{®}) ou le dioleyl tocopheryl methylsilanol (nom commercial Liposiliol C ^{®}), ainsi que les actifs cosmétiques liposolubles additionnels sont ensuite ajoutés, toujours à température ambiante, et homogénéisés avec l'ensemble pendant 10 minutes.

Les formulations cosmétiques F8 à F10 selon l'invention sont détaillées dans le tableau 3 ci-dessous :

**Tableau 3 : formulations cosmétiques F8 à F10 selon l'invention**

| | **Compositions quantitatives % m/m** | | |
|---|---|---|---|
| Constituants | F8 | F9 | F10 |

| **Vitamine C et silicium liposolubles** | | | |
|---|---|---|---|
| VC-IP^{®} (tétraisopalmitate d'ascorbyle) | 1,8 | 2 | 10 |
| Pro-DSB^{®} (dimethyl oxobenzo dioxasilane) | 0,5 | 0,7 | 1 |
| **Rapport molaire vitamine C / silicium** | **6,1** | **4,9** | **17** |

| **Huiles** | | | |
|---|---|---|---|
| Huile Colza | 24 | 10 | / |
| Huile Jojoba | 12 | 15 | / |
| Huile Macadamia | 30 | 15 | / |
| Huile de Nigelle | 10 | / | / |
| Oleine de karite | 20 | / | / |

| **Huiles estérifiées** | | | |
|---|---|---|---|
| Caprylic capric triglycerides | / | / | 50 |
| Coco-caprylate caprate | / | 34,2 | 23,6 |
| Dicaprylyl carbonate | / | 20 | 15 |

| **Ingrédient cosmétique additionnel** | | | |
|---|---|---|---|
| Tocophérol | 0,2 | 0,1 | 0,2 |

| **Actifs cosmétiques additionnels** | | | |
|---|---|---|---|
| Retinyl palmitate (vitamine A) | 0,2 | 0,35 | / |
| Tocophéryl acétate (Vitamine E) | 0,3 | 0,65 | / |
| Extrait de la fleur de Spilanthes acmella africaine (nom commercial Gatuline InTense ^{®}) | / | 2 | / |
| Coenzyme Q10 | / | / | 0,2 |

Les formulations cosmétiques F11 à F13 selon l'invention sont détaillées dans le tableau 4 ci-dessous :

**Tableau 4 : formulations cosmétiques F11 à F13 selon l'invention**

| | **Compositions quantitatives % m/m** | | |
|---|---|---|---|
| Constituants | F11 | F12 | F13 |

| **Vitamine C et silicium liposoluble** | | | |
|---|---|---|---|
| Cosphaderm AP^{®} (palmitate d'ascorbyle) | 1 | 5 | 5 |
| Pro-DSB^{®} (dimethyl oxobenzo dioxasilane) | 0,5 | 1 | / |
| Liposiliol C^{®} (dioleyl tocopheryl methylsilanol) | / | / | 6 |
| **Rapport molaire vitamine C / silicium** | **9,3** | **23,1** | **37,3** |

| **Huiles** | | | |
|---|---|---|---|
| Huile Colza | 20 | / | / |
| Huile de jojoba | 12 | / | 15 |
| Huile Macadamia | 20 | / | / |
| Huile de nigelle | / | / | 5 |
| Huile d'olive | / | / | 15 |
| Oleine de karite | 10 | / | / |

| **Huiles estérifiées** | | | |
|---|---|---|---|
| Caprylic capric triglycerides | 21 | 30 | 25 |
| Coco-caprylate caprate | / | 41 | 13,6 |
| Dicaprylyl carbonate | 14 | 20 | 15 |

| **Ingrédient cosmétique additionnel** | | | |
|---|---|---|---|
| Tocophérol | 0,1 | / | 0,2 |

| **Actifs cosmétiques additionnels** | | | |
|---|---|---|---|
| Retinyl palmitate (vitamine A) | / | 0,3 | / |
| Tocophéryl acétate (Vitamine E) | 0,4 | 0,7 | / |
| Extrait de Criste marine (nom commercial Native Essence) | 1 | / | / |
| Extrait de la fleur de Spilanthes acmella africaine (nom commercial Gatuline InTense ^{®}) | / | 2 | / |
| Coenzyme Q10 | / | / | 0,2 |

### Exemple 4 : Propriétés cosmétologiques des formulations cosmétiques selon l'invention.

Une comparaison d'activité a été faite entre :
- la formulation cosmétique F6 selon l'invention ; et
- une formulation cosmétique comparative FC1 ayant une composition quasi-identique, mais avec un rapport molaire entre la vitamine C liposoluble et le silicium liposoluble d'environ 1, et incorporant donc une concentration plus faible en vitamine C liposoluble.

Les deux formulations comparées sont détaillées dans le tableau 5 ci-dessous :

**Tableau 5 : formulations cosmétiques comparées**

| | **Compositions quantitatives % m/m** | |
|---|---|---|
| Constituants | F6 | FC1 |

| **Vitamine C et silicium liposolubles** | | |
|---|---|---|
| VC-IP ^{®} (tétraisopalmitate d'ascorbyle) | 1,5 | 0,3 |
| Pro-DSB^{®} (dimethyl oxobenzo dioxasilane) | 0,5 | 0,5 |
| **Rapport molaire vitamine C / silicium** | **5,1** | **1,02** |

| | **Huiles** | |
|---|---|---|
| Huile Tournesol | 40 | 40 |

| | **Huiles estérifiées** | |
|---|---|---|
| Caprylic capric triglycerides | 32,85 | 34,05 |
| Dicaprylyl carbonate | 25 | 25 |

| **Ingrédient cosmétique additionnel** | | |
|---|---|---|
| Tocophérol | 0,15 | 0,15 |

Ainsi les deux formulations cosmétiques comparées, à savoir F6 et FC1, ont été utilisées pendant 2 semaines (J0-J14), 2 fois par jour (matin et soir) par 5 personnes âgées de 40 à 70 ans, la formulation cosmétique F6 selon l'invention étant appliquée sur le côté droit du visage, la formulation cosmétique comparative FC1 sur le côté gauche du visage.

Les 5 personnes ont constaté un teint de peau plus éclatant dès les premiers jours d'utilisation sur le côté droit du visage.

Après 2 semaines d'utilisation bi-quotidienne, les 5 personnes ont constaté un teint plus uniforme du côté droit du visage, avec de plus une fermeté et une élasticité de la peau bien meilleure.

Pour mettre en évidence cette élasticité améliorée (corrélée au gain en fermeté), le test du « pincé de peau » de la joue (avec torsion et observation du retard de la peau à retourner à son état initial) a été réalisé à J0 et à J14 sur chaque joue.

Pour les 5 personnes, aucune différence entre les 2 joues n'était à noter à J0 alors qu'elles observent toutes un retour à la normale plus rapide et une peau qui reste moins marquée à J14 du côté droit par opposition à la peau du côté gauche.

## Revendications

1. Formulation cosmétique comprenant une phase lipophile, **caractérisée en ce que** ladite phase lipophile comprend :
- au moins une huile ;
- au moins un ester d'acide gras de vitamine C ; et
- au moins un précurseur liposoluble de silanol ;
le rapport molaire entre ledit au moins un ester d'acide gras de vitamine C et ledit au moins un précurseur liposoluble de silanol dans ladite phase lipophile étant supérieur ou égal à 1,5.

2. Formulation cosmétique selon la revendication 1, carcatérisée en ce que le au moins un ester d'acide gras de vitamine C est une une vitamine C liposoluble et le au moins un précurseur liposoluble de silanol est un silicium liposoluble.

3. Formulation selon la revendication 1, **caractérisée en ce que** le rapport molaire entre ledit au moins ester d'acide gras de vitamine C et ledit au moins un précurseur liposoluble de silanol dans ladite phase lipophile est supérieur ou égal à 2.

4. Formulation selon l'une quelconque des revendications précédentes, **caractérisée en ce que** la concentration molaire en l'au moins un ester d'acide gras de vitamine C est supérieure à 0,001 mol/l de ladite phase lipophile.

5. Formulation selon l'une quelconque des revendications précédentes, **caractérisée en ce que** la concentration molaire en l'au moins un précurseur liposoluble de silanol est supérieure à 0,0005 mol/l de ladite phase lipophile.

6. Formulation selon l'une quelconque des revendications précédentes, **caractérisée en ce que** ledit au moins un ester d'acide gras de vitamine C est choisi dans le groupe constitué du palmitate d'ascorbyle, du dipalmitate d'ascorbyle, du tétraisopalmitate d'ascorbyle, et de leurs mélanges.

7. Formulation selon l'une quelconque des revendications précédentes, **caractérisée en ce que** ledit au moins un précurseur liposoluble de silanol est choisi dans le groupe constitué du dioleyl tocopheryl methylsilanol, du dimethyl oxobenzo dioxasilane, et de leurs mélanges.

8. Formulation selon l'une quelconque des revendications précédentes, **caractérisée en ce que** l'au moins une huile est une huile choisie dans le groupe constitué des huiles naturelles, des huiles estérifiées, et de leurs mélanges.

9. Formulation selon l'une quelconque des revendications précédentes, **caractérisée en ce que** ladite phase lipophile comprend en outre au moins un ou plusieurs ingrédient(s) cosmétique(s) liposoluble(s) additionnel(s).

10. Formulation selon l'une quelconque des revendications précédentes, **caractérisée en ce que** ladite phase lipophile comprend en outre au moins un ou plusieurs actif(s) cosmétique(s) liposoluble(s) additionnel(s).

11. Formulation selon l'une quelconque des revendications précédentes, **caractérisée en ce que** ladite formulation est constituée par ladite phase lipophile et ladite phase lipophile est exempte d'eau.

12. Procédé de préparation d'une formulation cosmétique selon l'invention comprenant au moins les étapes suivantes de formation d'une phase lipophile :
1) on dispose d'au moins une huile, d'au moins un ester d'acide gras de vitamine C ou d'au moins une vitamine C liposoluble et d'au moins un précurseur liposoluble de silanol ou d'au moins un silicium liposoluble ;
2) l'au moins une huile, l'au moins un ester d'acide gras de vitamine C ou l'au moins une vitamine C liposoluble et l'au moins un précurseur liposoluble de silanol ou l'au moins un silicium liposoluble sont mélangés ;
le rapport molaire entre ledit au moins un ester d'acide gras de vitamine C ou ladite au moins une vitamine C liposoluble et ledit au moins un précurseur liposoluble de silanol ou ledit au moins un silicium liposoluble dans ladite phase lipophile étant supérieur ou égal à 1,5.

13. Utilisation d'une formulation cosmétique selon l'une quelconque des revendications 1 à 11, **caractérisée en ce qu'**elle est utilisée pour stimuler les fibroblastes.

14. Formulation cosmétique selon l'une quelconque des revendications 1 à 11, destinée à être utilisée pour stimuler les fibroblastes.

15. Kit cosmétique comprenant au moins :
- une formulation cosmétique selon l'une quelconque des revendications 1 à 11 ;
- un dispositif d'application de type roller.
